# EUROPEAN PATENT SPECIFICATION

(11) **EP 4 190 147 B1**
(45) Date of publication and mention of the grant of the patent: **25.06.2025**
(21) Application number: 21853173.9
(22) Date of filing: 30.07.2021
(51) Int. Cl.: A01K 15/02, G01N 33/00

(54) **TRAINING AID FOR TRAINING IN THE DETECTION OF TRIACETONE TRIPEROXIDE (TATP), PRODUCTION METHODS AND USE FOR DOG TRAINING**
TRAININGSHILFE ZUM TRAINIEREN DES NACHWEISES VON TRIACETONTRIPEROXID (TTP), HERSTELLUNGSVERFAHREN UND VERWENDUNG FÜR HUNDETRAINING
APPÂT POUR L'ENTRAÎNEMENT DANS LA DÉTECTION DE TRIPEROXYDE DE TRIACÉTONE (TAPT), PROCÉDÉS D'OBTENTION ET UTILISATION POUR L'ENTRAÎNEMENT DE CHIENS

(30) Priority: 03.08.2020 ES 202030829
(43) Date of publication of application: 07.06.2023
(73) Proprietor: Universidad del País Vasco/Euskal Herriko Unibertsitatea, 48940 Leioa (Bizkaia) (ES)
(72) Inventor: ISLA LOPEZ, Ainhoa, 48940 Leioa Bizkaia (ES); BARTOLOMÉ MORO, Luis Javier, 48940 Leioa Bizkaia (ES); COSTAS COUSO, María del Carmen, 48940 Leioa Bizkaia (ES); CASTRESANA PELAYO, José María, 48940 Leioa Bizkaia (ES); ETXEANDIA MUJIKA, Jon, 48940 Leioa Bizkaia (ES); ECHEVERRÍA MACHADO, Iñaki, 48940 Leioa Bizkaia (ES)
(74) Representative: Balder IP Law, S.L.
(86) International application number: PCT/ES2021/070581
(87) International publication number: WO 2022/029354

(56) References cited:
- US-A1- 2009 194 744
- US-A1- 2014 097 551
- US-A1- 2018 027 771
- ADEE SCHOON; SEBASTIAN GÖTZ; MARTIJN HEUVEN; MARTIN VOGEL; UWE KARST: "Training and Testing Explosive Detection Dogs in Detecting Triacetone Triperoxide", FORENSIC SCIENCE COMMUNICATIONS, vol. 8, no. 4, US , pages 1 - 7, XP009534888, ISSN: 1528-8005, Retrieved from the Internet <URL:https://archives.fbi.gov/archives/about-us/lab/forensic-science-communications/fsc/oct2006/research/2006_10_research01.htm> [retrieved on 20210430]
- SIMON ALISON, LAZAROWSKI LUCIA, SINGLETARY MELISSA, BARROW JASON, VAN ARSDALE KELLY, ANGLE THOMAS, WAGGONER PAUL, GILES KATHLEEN: "A Review of the Types of Training Aids Used for Canine Detection Training", FRONTIERS IN VETERINARY SCIENCE, vol. 7, XP055905293, DOI: 10.3389/fvets.2020.00313
- OXLEY JIMMIE C., SMITH JAMES L., MORAN JESSE, NELSON KEN, UTLEY WILLIAM E.: "Training dogs to detect Triacetone Triperoxide (TATP)", ALGORITHMS AND TECHNOLOGIES FOR MULTISPECTRAL, HYPERSPECTRAL, AND ULTRASPECTRAL IMAGERY XIX - PROCEEDINGS OF SPIE, SPIE, US, vol. 5403, 15 September 2004 (2004-09-15), US, XP055905292, ISSN: 0277-786X, ISBN: 978-1-5106-4548-6, DOI: 10.1117/12.555791

## Description

### TECHNICAL SECTOR

The present invention relates to triacetone triperoxide (TATP) baits for training in the detection of explosives, particularly, for canine units of the various bodies of law enforcement. Likewise, it also relates to production processes for producing these TATP baits and their use in training dogs.

### BACKGROUND

In recent decades, the use of improvised explosives in terrorist attacks has spread. Explosives of this type are homemade and relatively simple. Triacetone triperoxide (TATP) belongs to this group. The availability of its precursors (acetone, peroxides and acids), together with the ease of synthesis and the great detonation effect, attracts the interest of terrorists and is therefore an especially dangerous explosive. Accordingly, national and regional police officers from different countries train dogs in canine units to detect the scent of said explosive.

There are some terms and definitions in canine training that must be described for their correct interpretation, since they are of vital importance in the proper functioning of training baits. FALSE POSITIVE is defined when the dog marks "something" that does not correspond to the substance it should detect. This usually occurs due to the association of scents coming from, for example, the support on which the substance to be detected is impregnated, the additives added as stabilisers of the explosive, as well as any excess component during the manufacture of the explosive or any of its degradation products. In the same way, it is referred to as FALSE or FALSE NEGATIVE when the dog marks a position where there is nothing, or nothing similar that can be associated in any way with the substance to be detected. These errors are usually caused when the dog has great difficulty finding the trail of the explosive, making a marking that does not correspond in order to get a prize. Lastly, there is the case where the dog DOES NOT DETECT/PASSES BY and does not mark the position of a bait. This may be because the dog has not fixed the scent of the substance to be detected (training defect), that the conditions of the scenario (for example air currents) do not allow it to pick up the scent, or that the bait is not working correctly. As is to be expected, the lower the occurrence of false positives, false negatives or non-detections in training, the much higher the quality of training and the reliability of the dog's learning will be, this being what is desirable in view of the performance of the dog in the case of a real scenario.

There are different commercial baits on the market, but all of them have certain drawbacks such as creating false positives due to the presence of stabilising additives that are added for safe transport, the requirement of handling prior to its use and/or an excessive transport price.

Among these commercial baits there is one supplied by the US company TrueScent^{®}. This bait contains a small amount of TATP stabilised with acetonitrile as a solvent. Along with this bait, and in order to avoid false positives, blank baits, that is, baits that only include the stabilising solvent, are supplied to ensure that the dog detects the scent of the TATP and not the support/stabiliser. With this bait, training requires expensive dynamics and, in some cases, produces unreliable results as they produce false negatives and false positives.

TATP is sold in aqueous solution under the Gradko International Limited brand. This is because wet or damp TATP completely loses its explosive properties and its handling is completely safe. The drawback of this bait is that it has to be handled to get solid TATP, particularly, the aqueous solution of TATP must be subjected to a drying process before the bait can be used in training.

On the other hand, US companies ScentLogix^{™} and Gallant Technologies market non-explosive TATP priming charges. The problem with baits of this type is the high sale and transport price; in addition, canine units have a special interest in training their police dogs with explosives that have all of their properties.

Patent application US2018027771A describes a device for the training of explosives sniffer dogs comprising: an unspecified explosive assimilated by a solid (diatomaceous earth) or liquid carrier material, a housing having an interior space or a space including said interior space for receiving said carrier material with the explosive, a closure that allows the reversible gastight closure of the interior space or the space comprising said interior space, and a gas permeable device for preventing the carrier material from escaping from the interior space if it was not gas-tightly closed. This document does not refer to a specific explosive, particularly, it does not refer to TATP and, therefore, it is not possible to know if this device would be useful in training dogs for the detection of TATP without the need for any prior treatment before use. The device described in US2018027771A has several drawbacks for its use in training for the detection of explosives. First, the use of diatomaceous earth as an absorbent material requires the use of a housing to contain it, since this material is in powder form and, therefore, cannot be easily handled during training unless it is contained in some external container or housing. This device then turns out to be excessively bulky, so it presents difficulties when it comes to hiding it properly during training. As a result, the training is altered since the dog can detect the explosive by sight and not by smell alone. On the other hand, the need for a gastight closure or, alternatively, the incorporation of a permeable device that prevents the carrier material from getting out of the housing increases the production cost and complicates production.

In addition to the above, the inclusion of an external device (housing or container) to contain the internal support in which the explosive is located can give rise to significant drawbacks in the training for the detection of the explosive, such as:
- The housing only has an opening at the top, so the cone of scent only comes out at the top, causing a distortion in the training and high possibilities of false negatives or that the dog does not detect the device by smell.
- By including an external container, reusable for explosives of another type, there is the possibility of a memory effect between each test, as well as a high probability of false positives or low sensitivity in training tests (nondetection).
- Handling of the device to open and close it causes increased interference of human scent in the detection bait, this being a very important interference to avoid in the training of the detection of explosives by smell, since dogs can follow the human scent and not the scent of the explosive, causing false positives.

Patent application US2014097551A describes training aid materials for detecting homemade explosives such as TATP. As described herein, the method for producing this aid material comprises spreading an explosive powder on a porous material (glass microfibres or diatomaceous earth), storing this material with explosive in a container that facilitates sublimation of the explosive powder over time and the re-deposition thereof in powder form in the pores of the porous surface. In this procedure, sublimation takes place by heating, which can cause chemical alterations and degradation of TATP, thus modifying the proportions of TATP and its degradation by-products under normal conditions (temperature 25 °C and atmospheric pressure) in the bait used for training, which would lead to a reduction in the effectiveness of the training due to the increased possibility of false positives for the degradation products and not for the TATP explosive.

In patent applications US2014097551 A and US2018027771A, a porous material with a large surface area is sought to promote the deposition of solid explosive particles which, in the case of US2014097551A, are "artificially" sublimated by means of heating and which, when the temperature drops, are deposited along the chamber in which the sublimation has been carried out and where the supports (diatomaceous earth and glass fibre filter) are located. In the baits described in these documents, therefore, supports are necessary to maximise this deposition of solid particles.

On the other hand, patent US9784723B describes a method for preparing a (non-detonable) explosive vapour source and encapsulating it in microspheres or microcapsules of a polymer such as, for example, polystyrene or polysulfone, which retains the vapour until heat is applied to release the explosive vapour. Accordingly, the bait described in this US patent requires handling of the bait prior to use, which is a drawback for carrying out the training of canine units for the detection of explosives.

Patent application US 2009/194744 A1 discloses a scent simulant of a chemical explosive material, such as TATP, wherein cellulose acetate butyrate may be used as binder.

### BRIEF DESCRIPTION OF THE INVENTION

As seen above, there are commercial products for training in the detection of explosives, and several prior art documents describing products or devices of this type have also been found. However, the baits for training in the detection of explosives available to date have certain drawbacks, particularly, they can generate false positives, false negatives or non-detections by dogs, thus reducing their effectiveness; they require handling prior to use, which complicates training; they have an excessive manufacturing and/or transportation cost. Furthermore, some of the devices described above have an external casing that increases their volume and, accordingly, alters the training of dogs in the detection of the explosive by smell alone. The present invention presents notable differences with previously disclosed baits and, as a result of these differences, allows the technical problems mentioned above to be overcome or minimised.

In particular, commercial products are generally stabilised with a solvent or additive with a characteristic scent that can lead to false positives. In the baits of the present invention, the molecule used is TATP without any type of transformation, passivation or stabiliser, so false positives are minimal.

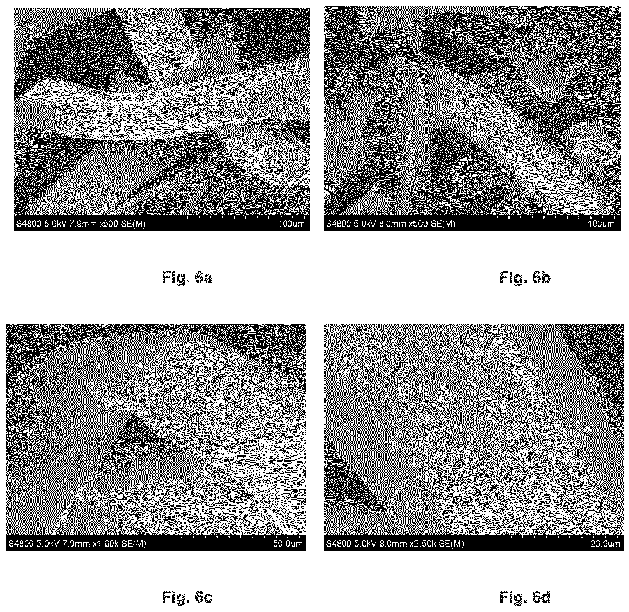

Another important advantage of the TATP baits of the present invention is that they comprise such a small amount of explosive, a maximum of 0.05 g of TATP, preferably a maximum of 0.03 g of TATP, and more preferably a maximum 0.02 g of TATP, that there is no risk of explosion in handling during training, or during the course of its synthesis as described in this patent application. In particular, for baits comprising solid TATP adsorbed on the support made of cellulosic material (also referred to herein as Hauts-TATP baits), the amount of TATP is preferably between 50 mg and 0.5 mg, more preferably between 20 mg and 0.5 mg of adsorbed solid TATP. The tests carried out (see Figures 6a-6d below) show that the solid TATP particles are adsorbed on the fibres of the cellulosic material, in particular cellulose acetate, providing a strong enough interaction to allow handling of the baits without the adsorbed solid TATP dislodging.

For baits with TATP vapour adsorbed on a support (also referred to herein as Hodei-TATP baits), there is no solid explosive, but rather just the vapour from this explosive. This fact facilitates use of this training bait and its transport to the different training areas of the different police forces, not only regional but also national or international police forces. The transport of this baits does not entail any extra danger due to an explosive material, which facilitates its national and international commercialisation, considerably increasing the profit margin by cutting tariff rates.

In addition, the TATP baits object of the present invention are simpler than those described in the prior art, which entails significant advantages for their manufacture and use for training in the detection of the TATP explosive. One of the objectives of the present invention is to minimise the complexity of the baits as much as possible, that is, to reduce the materials that comprise the training bait, while maintaining the necessary properties to maximise the applicability of the baits and achieve efficient training. More specifically, the solid support included in the bait described herein is preferably a single solid piece, which allows it to be easily handled, for example, with inert tweezers, without the need for an external housing to contain it. This feature of the baits of the present invention represents an advantage over other types of baits, such as, for example, those described in US2018027771 A, where the support used (diatomaceous earth or ionic liquid) requires an external housing, which results in a greater complexity and volume of the bait for training in the detection of explosives.

The present invention also provides a simple and effective method of producing TATP baits for training in the detection of this type of explosive, where the amount of TATP used both during the process for producing the bait and that present in the bait itself is lower than that described above. The invention described herein, therefore, allows to considerably reduce the economic cost of baits, particularly, in the case of manufacturing baits comprising the aroma of TATP (Hodei-TATP baits).

In particular, the invention provides a simplified procedure for producing TATP baits for training in the detection of explosives, wherein the adsorption of TATP on the solid support comprising a cellulosic material can take place directly from solid TATP. Thus, in the method of manufacturing baits comprising solid TATP as described herein, this adsorption can be carried out directly at the time of filtering an aqueous suspension of TATP, thus shortening the manufacturing steps and providing a simpler process than those described in the prior art.

In other particular embodiments of the invention, the solid TATP and the support made of cellulosic material are placed in the same closed container without contact between the two solids, so that the TATP vapours are adsorbed directly on the support that is part of the bait to be used in training. By avoiding a heating process to cause sublimation of the explosive, possible chemical alterations and degradation to the corresponding TATP by-products (acetone, DADP) are prevented, so that the vapours adsorbed on the support made of cellulosic material correspond to TATP and, to a lesser extent, to its degradation products in the usual degradation percentages at atmospheric pressure and a temperature between 10 °C and 35 °C, preferably between 20 °C and 25 °C.

Unlike US9784723B, the bait for training in the detection of explosives of the present invention does not comprise explosive vapour encapsulated in a plastic polymer and, furthermore, no heat is applied to release the explosive vapour from the bait. Accordingly, the present invention provides a training aid material for the detection of explosives that can be used by means of direct exposure, without the need for any additional treatment, since the aroma of TATP is progressively released from the support at room temperature, that is, at the temperature at which training takes place, particularly, between 10 °C and 35 °C, preferably between 20 °C and 25 °C.

In summary, by containing very small amounts of solid TATP (Hauts-TATP baits) or simply TATP vapour (Hodei-TATP baits), and since it is not necessary to apply temperature to achieve sublimation of the explosive, since this is carried out at atmospheric pressure and a temperature between 10 °C and 35 °C, preferably between 20 °C and 25 °C, a bait for training in the detection of TATP that is safer both in synthesis and in use is produced. Likewise, since no type of solvent or stabiliser that can give off volatile compounds is used, the number of false positives is considerably reduced. The solid support comprising a cellulosic material, preferably acetylated cellulose, cellulose, cellulose butyrate or a combination of the above, can be a single piece with the desired dimensions, so it is not necessary to use any housing containing said solid support. Thus, the baits according to the present invention in which the explosive, either in solid form or as a vapour, is adsorbed on a single piece of solid cellulosic material can be used directly for training in the detection of explosives, reducing the human handling (opening, closing, reusing, etc.) that would take place if it were necessary to contain the bait in some type of housing or external container. This is an important advantage, since it prevents dogs from following the human scent and, at the same time, the size of the device is minimised, which allows it to be hidden better and thus prevents dogs from detecting the bait by sight.

Likewise, another advantage provided by the bait for training in the detection of explosives of the present invention in which the solid support comprises a cellulosic material that is acetylated cellulose (also referred to as "cellulose acetate") is that the TAPT is released gradually from this support, allowing the prolonged use over time and, particularly, the reuse of the bait in the training in the detection of explosives.

The different embodiments of the bait described herein are easy to use by the user and do not require any type of additional training or any type of subsequent handling.

### DETAILED DESCRIPTION OF THE INVENTION

In a first aspect, the present invention relates to a bait for training in the detection of explosives, in particular triacetone triperoxide (TATP), characterised in that it comprises a maximum of 0.05 g of TATP adsorbed on a solid support comprising a cellulosic material, wherein the bait preferably comprises a maximum of 10% by weight of water, more preferably a maximum of 7% by weight of water, even more preferably a maximum of 5% by weight of water, said percentage being expressed by weight with respect to the total weight of the bait.

In that sense, the present invention relates to a TATP bait, also referred to as training bait, training aid material, or training material, designed for training in locating TATP explosive, especially for dogs by detecting odorous compounds present in the vapours that this explosive gives off.

These TATP baits contain a maximum of 0.05 g of TATP, preferably a maximum of 0.03 g of TATP, and more preferably a maximum of 0.02 g of triacetone triperoxide (TATP), adsorbed on a solid support made of cellulosic material, preferably the support is a single piece and, more preferably, a single piece with a volume between 100 mm³ and 2300 mm³. Due to the low amount of TATP contained in these baits, safety during their manufacture, storage, transport and use is significantly increased. In particular, the aid materials for training in the detection of explosives described herein can be used safely, both for the animals and for the personnel carrying out the training.

In the TATP baits according to the present invention, the TATP (solid or gas) is adsorbed on the cellulosic material comprised in the solid support, that is, the solid or vapour TATP molecules are retained on the porous surface of the support.

Unlike commercial dog training aid materials for the detection of explosives, where the TATP is deposited on the solid support mixed with stabilising substances, particularly, water or organic solvents such as acetonitrile or ethyl acetate, in the baits of the present invention, only TATP (solid or vapour) is adsorbed on the solid support made of cellulosic material. In that sense, the TATP priming charges described herein preferably contain a maximum of 10% by weight of water, more preferably a maximum of 7% by weight of water, even more preferably a maximum of 5% by weight of water, said percentages being expressed by weight with respect to the total weight of the bait.

The water content present in TATP baits can be determined by means of thermogravimetry, for example, using a thermobalance. To that end, a representative part of the TATP bait sample to be analysed can be heated on a thermobalance with a temperature gradient of 10 °C/min starting at 25 °C up to 500 °C. The water content corresponds to the percentage of weight loss with respect to the initial weight of the sample of the bait used to carry out the analysis at a temperature of 105 °C. The selected guidelines are adjusted to usual parameters in the measurement of moisture in solids by means of this type of scientific instrumentation.

In the case of water, in addition to stabilising the TATP, it eliminates its explosive properties. This effect is something unwanted for dog trainers, since they generally want guarantees that the molecule with which they are training is explosive. To that end, the water content in the TATP baits of the present invention is preferably a maximum of 10% by weight of water, more preferably a maximum of 7% by weight of water, even more preferably a maximum of 5% by weight of water, with respect to the total weight of the bait. According to verifications carried out within the framework of the present invention, this water content is not sufficient to mitigate the explosive capacity of the bait. In the case of commercial water-stabilised baits mentioned in the background, these are suspensions of the explosive in aqueous solutions in which the percentage of water is much higher (>1000% water with respect to the weight of TATP in the bait) than what is handled herein.

As mentioned above, the support comprised in the bait of the present invention is a solid cellulosic material, "cellulosic material" being understood to be one that comprises, as its main component, cellulose, acetylated cellulose, cellulose butyrate or another type of modified cellulose, as well as a combination of the above. This support is inert, porous and, in addition, allows adsorption of TATP, as well as its subsequent desorption, without any additional treatment. Thus, the bait described herein can be used easily and effectively for training in the detection of the TATP explosive, since no type of treatment prior to its use is required, which is a significant advantage in terms of safety, speed and effectiveness of said training while ensuring that dogs are trained with the synthesised TATP molecules, without having carried out a subsequent sublimation/deposition process that could generate physicochemical alterations in the explosive.

"Inert" is understood to mean in the present invention any material that is not chemically reactive, in particular in the conditions in which the manufacture of the baits takes place, as well as during the use thereof in training.

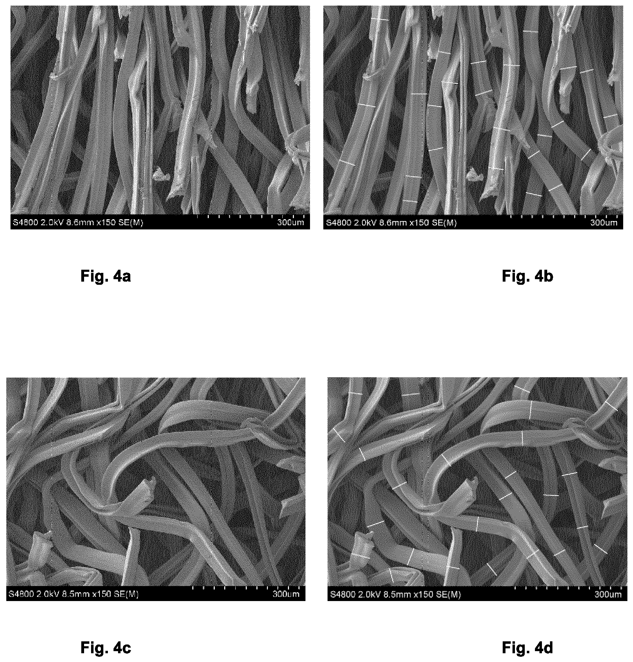

"Porous" is understood to mean in the present invention any material that contains a % of void volume with respect to the total volume of the material. In particular, the cellulosic materials comprised in the baits are porous materials made up of a multitude of intertwined fibres that give rise to a high surface area (see Figures 4a-4d). These supports made of cellulosic material can have a minimum specific surface area of 0.08 m²/g, preferably between 0.16 m²/g and 0.32 m²/g. The specific surface area can be determined by techniques known in the art.

In particular, the specific surface area can be determined from the calculation of the total surface area. In that sense, for example, the surface area indicated above corresponds to a minimum total surface area of 56 cm², preferably between 56 and 225 cm², for a cylindrical support with a weight of 70 mg on average. The total surface area can be determined, for example, by calculating the density of fibres in the support from photographs obtained by scanning electron microscope (SEM) and calculating the size of the fibres of said support from the diameter and length of said fibres.

An important feature of the cellulosic material, in particular acetylated cellulose, used as a solid support in the baits of the present invention is its ability to adsorb and desorb gaseous TATP molecules as a function of TATP vapour pressure (P_{vTATP}). This property of reversible TATP adsorption/desorption at a temperature between 10 °C and 35 °C, preferably between 20 °C and 25 °C, is not one that all porous or polymeric materials have. In that sense, for example, compounds such as polydimethyl-polysiloxanes (PDMS) or carboxenes (CAR) are not valid support materials for the present invention because, although they are very efficient in their adsorption process, the aroma of TATP adsorbed on same is usually not desorbed unless the adsorbent is heated above 50 °C, making its use as a support in baits for training in the detection of explosives unfeasible, since it would be necessary to heat the baits during training so that it gave off the aroma. In a particularly advantageous manner, the cellulosic material used as a support in the baits of the present invention is capable of adsorbing the aroma of the explosive in a reversible manner, that is, when the cellulosic material is in the presence of TATP at a vapour pressure of this compound (P_{vTATP}) between 1.06 x 10⁵ Pa and 1.42 x 10⁵ Pa, the cellulosic material adsorbs TATP in the form of a gas (also referred to herein as "vapour" or "aroma"), while during dog training, when the baits is at a temperature between 10 °C and 35 °C, preferably between 20 °C and 25 °C, and a P_{vTATP} less than the range indicated above, the support desorbs the TATP.

In addition to the properties indicated above, the cellulosic material comprised in the support of the baits described herein, preferably acetylated cellulose, cellulose, cellulose butyrate or a combination of the above, has the additional advantages of being a non-toxic material for dogs or trainers, is inexpensive, light and able to form a solid support in a single piece (not particulate), thus facilitating the handling of the **TATP** baits during training, for example, with inert tweezers, and avoiding having to place the support in a housing or external container as would occur in the case of being a particulate support.

As mentioned above, the cellulosic material can be cellulose, acetylated cellulose, cellulose butyrate or a combination of the above. **In** the context of the present invention, "acetylated cellulose" is to be understood as referring to an acetate ester of cellulose, therefore it can also be referred to as "cellulose acetate". Depending on the degree of acetylation of the cellulose hydroxy groups, the acetylated cellulose can be cellulose (mono)acetate, cellulose diacetate or cellulose triacetate. Preferably, the acetylated cellulose is cellulose (mono)acetate.

Although the main component of the support is a cellulosic material, the support of the present invention may comprise other non-odorous additives such as, for example, titanium oxides, calcium oxide or zinc oxide, in addition to a polymeric compound with hydrocarbon bonds. In particular, the cellulosic material comprised in the training baits of the present invention may be tobacco filters (cellulose acetate).

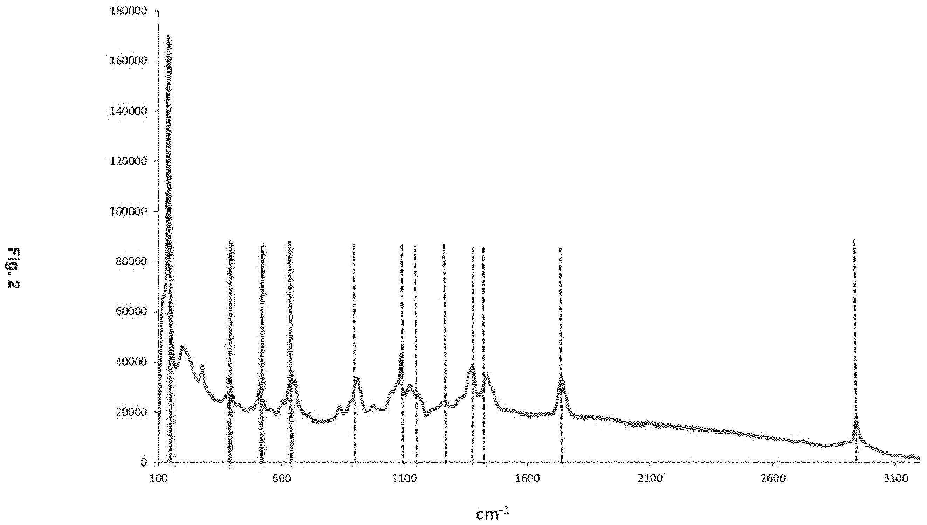

Figure 2 shows the Raman spectrum of a support according to particular embodiments of the present invention wherein the cellulosic material is cellulose (mono)acetate. In this spectrum, the characteristic bands of cellulose acetate (dashed line) can be easily distinguished, and the presence of the characteristic bands of titanium oxide (anatase) can also be observed (continuous line). This inorganic component is commonly found in cellulosic materials of this type, since it is used for bleaching in the industrial manufacturing process.

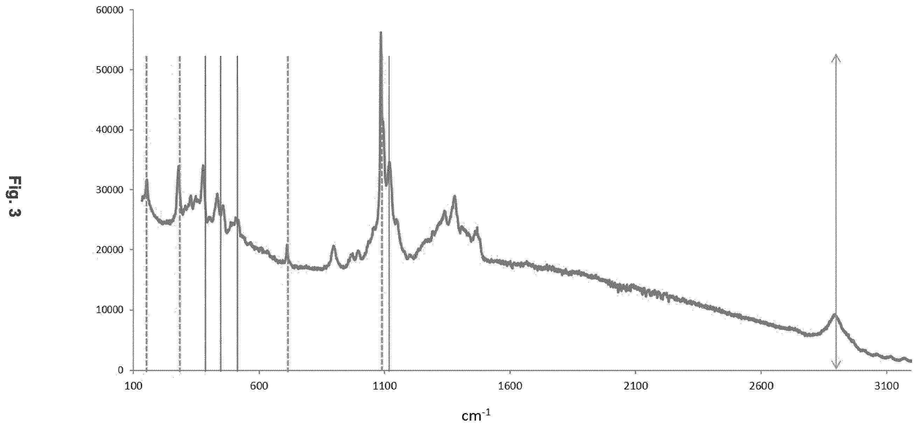

In addition, the solid support made of cellulosic material according to particular embodiments of the present invention may comprise a polymeric material partially coating the solid support. In particular, when the solid support is a cylinder such as, for example, a tobacco filter, this polymeric material can be an organic hydrocarbon material present on the perimeter of the cylinder, but not at its bases. As can be observed in Figure 3, the polymeric material characterised by bands of an organic hydrocarbon material (line with the ends ending in arrows in Figure 3) may further comprise traces of calcite (CaO) and zincite (ZnO), shown by a solid line in this figure.

The solid support comprising cellulose acetate according to particular embodiments of the present invention may be made up of a multitude of intertwined fibres, as can be seen in the photographs obtained by means of scanning electron microscopy (see Figures 4a - 4d). According to these embodiments, the fibres can have an average diameter of 42.4 microns, with 95% being between 39 and 47 microns (25 measurements at two different points n_{TOTAL}=50).

The solid support used in the present invention can have different shapes and dimensions. In particular, this support can be cylindrical and have a length of 10 mm to 20 mm, particularly, it can have a length of 15 mm. Likewise, the cylindrical support can have a diameter between 4 mm and 8 mm, particularly, it can have a diameter of 6 mm. Accordingly, said support can be a single piece with a volume preferably between 100 mm³ and 2300 mm³, more preferably 1700 mm³. If the support has a size larger than 20 mm, the training bait comprising said support is detected by the dogs by sight instead of by smell and, therefore, is not useful for the detection of explosives by smell. On the other hand, if the support is excessively small, in particular less than 10 mm, the surface may not be sufficient for a sufficient amount of TATP to be adsorbed, which could make it difficult for sniffer dogs to locate the baits, giving rise to the occurrence of non-detections or false negatives. These solid supports can also be circular with volumes comparable to those used in their cylindrical version. This implies the use of filters with a diameter between 4 mm and 8 mm. In the same way, it can be extrapolated to any type of geometry in the filter that complies with the described working volume, that is, between 100 mm³ and 2300 mm³, particularly, 1700 mm³.

In particular embodiments, the TATP bait for training in the detection of explosives consists of triacetone triperoxide (TATP) and, optionally, one or more of its synthesis by-products or degradation products, on a solid support made up of cellulosic material, preferably cellulose, acetylated cellulose, cellulose butyrate or a combination of the above, preferably with a maximum water content of 10% by weight of water, more preferably a maximum of 7% by weight of water, even more preferably a maximum of 5% by weight with respect to the total weight of the bait (W_{H2O}/W_{BAIT}).

According to the present invention, TATP degradation products are diacetone diperoxide (DADP), acetone and, to a lesser extent, tetraacetone tetraperoxide (TeATeP). The presence of these compounds occurs naturally when TATP breaks down into DADP and acetone, or when TeATeP is created as a dimer in the synthesis process. Knowing that the maximum amount of TATP in the bait is 50 mg, if the TATP were completely degraded, the maximum amount of each of the degradation products would be 30 mg for DADP and 13 mg of acetone. However, taking into account that the degradation calculated in the tests carried out by means of semi-quantitative analysis of the by-products was 1%, the amounts of by-products can be about 0.01 mg of DADP and 0.005 mg of acetone. In the case of TeATeP, this by-product has not been detected in any of the analyses in any bait, so its presence is considered residual or trace (<0.1 mg). The following table 1 shows the maximum amounts of mass of each of the most common components in the training bait.

**Table 1. Amounts of each of the compounds in the training bait under normal conditions of T and P, that is, 25 °C and 1 atm.**

| **Description of compound** | **Max. content by weight (mg)** |
|---|---|
| **TATP** | 50 |
| **DADP** | 30 |
| **Acetone** | 13 |
| **TeATeP** | 0.1 |

These degradation products can be analysed by means of gas chromatography, for example, in an Agilent Technologies 5975C gas chromatograph (Agilent Technologies, Avondale, CA, USA). To carry out this analysis, it is possible to use a column that is 30 m long, 0.25 mm in diameter and 0.25 µm of film and is of the same brand as the chromatograph used. Chromatographic conditions can be as follows: an injection temperature of 120 °C with a split injection mode (Split ratio 100:1), a desorption time of 15 minutes, and helium flow of 1.3 ml/min. The oven temperature program used can be as follows: initial temperature of 50 °C, hold constant for 2 minutes, to continue with a ramp of 10 °C/min until reaching 90 °C, at which temperature it is maintained for 3 minutes. Then another ramp of 20 °C/min can be carried out up to 200 °C, and once the final temperature is reached, it is maintained for 2 minutes. Total analysis time is 16.5 minutes.

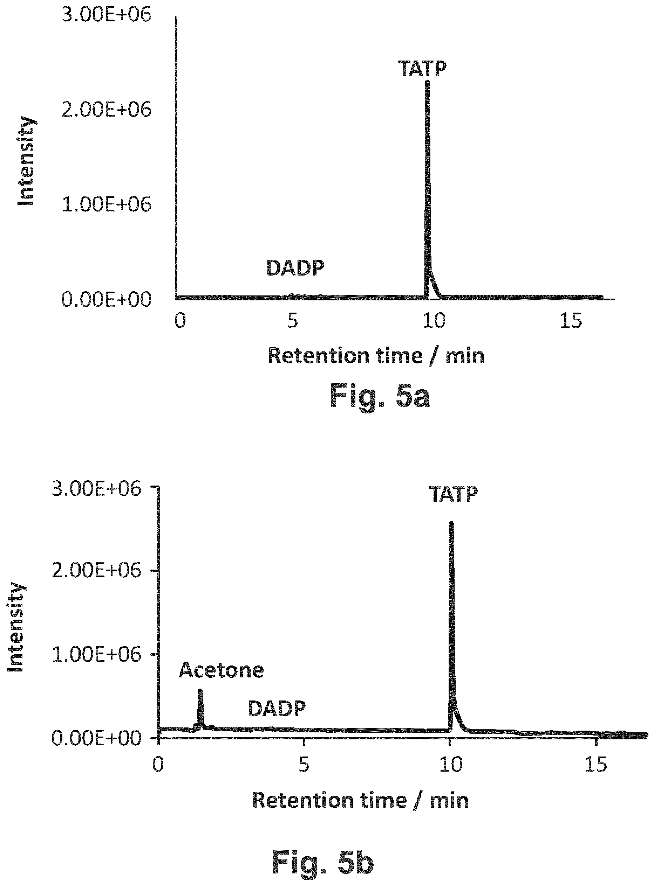

Under the conditions described above, in the case of Hauts-TATP baits, the chromatographic signal of TATP appears at minute 9.7 with a very high intensity, a compound identified by the NIST2017 spectral library with a confidence percentage greater than 90%. In addition, at minute 4.5 and with an intensity ratio with respect to TATP of 1:100, another chromatographic signal identified as diacetone diperoxide (DADP) is obtained with the same percentage of confidence (Figure 5a). In the case of Hodei-TATP baits (Figure 5b), the chromatographic signal of acetone is observed at minute 1.9, the DADP signal is observed at minute 4.5, and the TATP signal is observed at minute 9.7, where the intensity ratio is 39.5:1:100 (acetone: DADP: TATP).

In particular embodiments of the invention, the bait described herein (also referred to herein as Hauts-TATP bait) may comprise 50 mg to 0.5 mg of solid TATP adsorbed on the solid support made of cellulosic material, preferably 20 mg to 0.5 mg of solid TATP, a particular example of this embodiment is the TATP bait as described herein, comprising 20 mg of solid TATP adsorbed on a cylindrical acetylated cellulose filter measuring 15 mm long and 6 mm in diameter.

In additional particular embodiments, in the bait of the present invention (also referred to herein as Hodei-TATP bait), the TATP is adsorbed in vapour form on the support comprising a cellulosic material. In that sense, in another particular example of the present invention, the bait for training in the detection of the TATP explosive comprises TATP vapour adsorbed on a cylindrical acetylated cellulose filter measuring 15 mm long and 6 mm in diameter. The TATP content in the Hodei-TATP bait is preferably in the range of 20 µg to 100 µg TATP.

In a second aspect, the present invention relates to a process for obtaining the bait for training in the detection of explosives described herein (particularly the bait referred to as Hauts-TATP), characterised in that it comprises:
a) obtaining an aqueous suspension of TATP, and
b) adsorbing the TATP present in the aqueous suspension of step a) on a solid support comprising a cellulosic material, preferably cellulose, acetylated cellulose, cellulose butyrate or a combination of the above; more preferably acetylated cellulose.

The solid support where the solid TATP present in the aqueous suspension of step a) is adsorbed is preferably a single piece of cellulosic material with the appropriate size to adsorb a maximum of 0.05 g of TATP, preferably between 50 mg and 0.5 mg, and more preferably between 20 mg and 0.5 mg of TATP. This single piece of cellulosic material can have any geometric shape, where its volume is preferably between 100 mm³ and 2300 mm³, particularly, 1700 mm³.

To carry out step b) of adsorption of the solid TATP present in the aqueous suspension on the solid support, the aforementioned aqueous suspension of TATP is brought into contact with the solid support, for example, placing one or more supports made of cellulosic material in the solid collection part of a solid-liquid filtration system such as a Büchner funnel or equivalent system. In particular, to carry out this adsorption process, a glass wool filter can be placed on a Büchner funnel. It is possible to place on this filter one or more supports made of cellulosic material, preferably acetylated cellulose. Next, the aqueous suspension of TATP can be added onto the supports placed in the Büchner funnel. After the suspension is filtered, cleaning with water can be done to remove any trace of acid that may remain. In preferred embodiments, the system can be left filtering for an hour, preferably half an hour, to dry the TATP bait, thus being able to produce TATP baits with a maximum water content of 10% by weight of water, more preferably a maximum of 7% by weight of water, even more preferably a maximum of 5% by weight of water with respect to the total weight of the bait (measured by thermogravimetry, in particular according to the conditions indicated hereinabove). Once the TATP has been adsorbed on the supports made of cellulosic material, the baits produced can be kept, for example, in a screw-top glass vial, in freezer until use, preferably at a temperature between -18 °C and -25 °C.

In particular embodiments of the present invention, the aqueous suspension of TATP is obtained by means of the following steps:
a-i) mixing acetone and hydrogen peroxide at a temperature between 3 and 16 °C, preferably 9 °C;
a-ii) adding acid to the mixture obtained in a-i) for a time between 10 and 30 minutes, preferably 15 minutes;
a-iii) stirring the mixture of a-ii) for at least 5 hours, preferably from 5 hours to 24 hours, more preferably 5 hours, obtaining triacetone triperoxide (TATP); and
a-iv) suspending the triacetone triperoxide (TATP) of step a-iii) in water, obtaining the aqueous suspension of TATP;
wherein the stoichiometric ratio of acetone:hydrogen peroxide:acid is 3:3:1, and the amount of acetone is less than 10 mmol, more preferably equal to or less than 5 mmol, it being particularly preferable to use an amount of acetone equal to 2.7 mmol.

Different acids can be used in the TATP production process described herein. In particular, inorganic acids such as, for example, sulfuric acid, hydrochloric acid or nitric acid, can be used. These acids are readily available commercial products. Preferably, the acid used is sulfuric acid and, particularly, 98% w/w sulfuric acid. The use of sulfuric acid in the process described herein allows TATP to be produced without by-products that may affect the detection of odorous vapours during training, in less time, particularly, under the preferred conditions of the TATP production process of the present invention, the reaction can be completed in a period of 5 hours.

In a third aspect, the present invention relates to a process for obtaining the bait for training in the detection of explosives described herein (particularly, the bait referred to as Hodei-TATP), comprising keeping solid TATP and a solid support comprising a cellulosic material, preferably cellulose, acetylated cellulose, cellulose butyrate or a combination of the above, more preferably acetylated cellulose, in the same closed container for a minimum period of 7 days, preferably from 7 days to 30 days, without contact between the two solids. The TATP content in the bait obtained by this procedure is preferably in the range of 20 µg to 100 µg of TATP.

The process for obtaining the baits that comprise TATP in vapour form (Hodei-TATP baits) described in this patent application takes place at room temperature and ambient pressure, that is, without applying any type of pressure or temperature that modifies the conditions of the place where the process takes place. In particular, this process can take place at a pressure between 1.06 x 10⁵ Pa and 1.24 x 10⁵ Pa, preferably at 1.1 x 10⁵ Pa, and a temperature between 10 °C and 35 °C, preferably between 20 °C and 25 °C.

In that sense, in the process for obtaining Hodei-TATP baits, the TATP sublimation process does not take place by raising the temperature, but rather the system is allowed to reach equilibrium (solid/gas) at the temperature mentioned above, and the TATP vapour that is generated is adsorbed on the cellulosic material.

Preferably, the Hodei-TATP baits obtained according to the process described herein are kept packaged after the adsorption period, to be used directly in training dogs in the detection of explosives.

In particular embodiments of the present invention, the maximum amount of solid TATP present in the closed container is 200 mg to 6 mg and the distance between the solid TATP and the solid support is 3 cm to 10 cm. The container where this step takes place is preferably long and narrow, particularly, when the supports have a cylindrical shape and a length between 20 mm and 10 mm. In particular embodiments of the present invention, the container is 1 cm to 5 cm wide, and 10 cm to 20 cm long. The volume of the container used is 38 cm³, and the internal area is 47.50 cm². The ratio of the area of the container used and the grams of TATP in the container is between 7.92 cm²/mg and 0.23 cm²/mg.

The containers may have other geometries suitable for the arrangement of the supports and the explosive without touching each other with the volumes suitable for contact between the vapour of the explosive and the support, that is, having a volumetric capacity between 8 cm³ and 400 cm³. Under these conditions, between 1 and 6 Hodei-TATP baits for training in the detection of explosives can be obtained. This container can be made of glass or any other material not permeable to TAPT vapour. In the process described herein, this container must be closed to achieve the necessary pressures, although it is not necessary for this seal be gastight.

In particular embodiments of the present invention, solid TATP is obtained by means of the following steps:
i) mixing acetone and hydrogen peroxide at a temperature between 3 and 16 °C, preferably 9 °C;
ii) adding acid to the mixture obtained in a) for a time between 10 and 30 minutes, preferably 15 minutes;
iii) stirring the mixture of ii) for at least 5 hours, preferably from 5 hours to 24 hours, more preferably 5 hours, producing solid triacetone triperoxide (TATP);
wherein the stoichiometric ratio of acetone:hydrogen peroxide:acid is 3:3:1, and the amount of acetone is less than 10 mmol, preferably equal to or less than 5 mmol, more preferably equal to or less than 2.7 mmol.

In addition to steps i) to iii) mentioned above, the obtaining process that is described herein may comprise the following steps:
iv) suspending the triacetone triperoxide (TATP) of step iii) in water, obtaining the aqueous suspension of TATP; and
v) filtering the suspension of step iv).

As a result of step v), solid TATP is recovered again. The inclusion of these additional steps in the process for obtaining solid TATP makes it possible to obtain a product of greater purity, in those cases in which the solid TATP obtained after the reaction contains traces of acid. However, in other embodiments these steps can be carried out to collect the TATP more safely from the container where it has reacted.

Different acids can be used in the process for obtaining TATP described herein. In particular, sulfuric acid, hydrochloric acid or nitric acid can be used. Preferably, the acid used is sulfuric acid and, particularly, 98% w/w sulfuric acid. As mentioned above, the use of sulfuric acid under the conditions established herein allows TATP to be obtained without by-products that may affect the detection of odorous vapours during training in less time, particularly, in 5 hours.

Step v) can be performed by placing a glass wool filter in a funnel and filtering the TATP through it. Subsequently, the solid TATP can be collected with a spatula and gradually transferred to the container where the manufacture of the bait will take place, for example, a tube as described herein.

The present invention also relates to baits for training in the detection of explosives that are or that can be obtained by any of the procedures described herein.

In a fourth aspect, the present invention relates to a use of TATP baits that are described herein for the training of dogs, preferably police dogs, in the detection of TATP explosives.

In particular, the baits of the present invention can be used directly for training dogs in the detection of TATP explosives without the need for any prior handling that alters the olfactory profile of the TATP explosive, since the desorption of TATP (solid or gas) from cellulosic material can be carried out at atmospheric pressure and a temperature between 10 °C and 35 °C, preferably between 20 °C and 25 °C, without the need to submit the bait to heating steps or other treatments that accelerate desorption.

Potential applications are related to training police dogs for the detection of explosives of this type. In particular, users and potential buyers of the bait described herein are basically all the canine units of the different bodies of law enforcement of a country, for example, the Spanish regional police forces (Ertzaintza, Navarre Provincial Police, Mossos d'esquadra), the National Police or other bodies of law enforcement, such as the Civil Guard or the Royal Guard.

In the case of baits with TATP vapour adsorbed on a support comprising a cellulosic material, it is possible that for them to be marketed in a country other than the one in which they were manufactured, since the transport of this type of baits or training aid material does not require any special treatment or security measure.

### BRIEF DESCRIPTION OF THE FIGURES

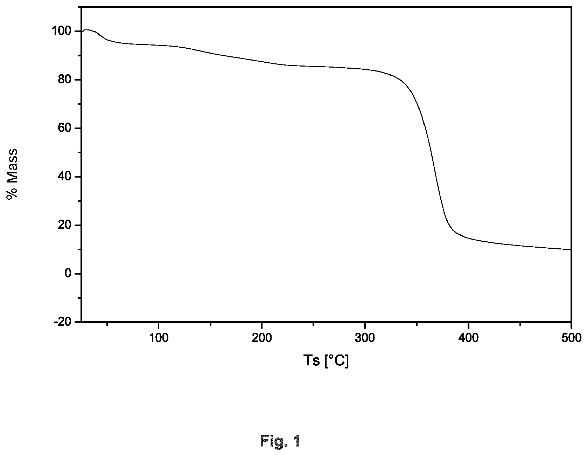

Figure 1. Thermogram for a bait loaded with TATP (replicate 2). It is possible to see the drop in weight of the bait as temperature increases: For this replicate (T=105 °C, W_{H2O}/W_{BAIT}: 5.9%).
   This analysis was repeated three times under the same conditions, resulting in a water content (W_{H2O}/W_{BAIT}) of 5% by weight with respect to the initial weight of the TATP bait.
Figure 2: Raman spectrum of cellulosic materials that can be used as a solid support in some embodiments of the TATP baits of the present invention. This figure shows the RAMAN spectrum of the support made of cellulose acetate without the adsorbed TATP explosive. The dashed line shows the characteristic signals of cellulose acetate, while a continuous line shows those of titanium oxide, based on the information provided by the databases: "Renishaw minerals and inorganic materials database" and "Renishaw polymeric materials database".
Figure 3: Raman spectrum of polymeric material coating the perimeter of cellulosic materials of cellulose acetate that can be used as a solid support in some embodiments of the TATP baits of the present invention. This figure shows the RAMAN spectrum of the perimeter of a support without the adsorbed TATP explosive. The dashed line shows the characteristic signals of calcium oxide and the continuous line shows those of zinc oxide. A line with the ends ending in an arrow shows the characteristic band of the polymeric material coating the perimeter. Identification made based on the information provided by the databases: "Renishaw minerals and inorganic materials database" and "Renishaw polymeric materials database".
Figures 4a - 4d: Scanning electron microscope (SEM) photographs of different parts of the solid support (without TATP). The measurements taken of the diameter of the cellulose acetate fibres are shown in yellow.
Figure 5: Representative chromatograms in SCAN mode of the Hauts-TATP baits (Fig. 5a) and the Hodei-TATP baits (Fig. 5b).
Figures 6a - 6d: Scanning electron microscope (SEM) photographs of different parts of the Hauts-TATP support according to particular embodiments of the present invention.
Figure 7: Raman spectrum of the base of the cylinder-shaped support included in the Hauts-TATP baits according to particular embodiments of the present invention. The characteristic signals of TATP are shown in a dashed line, the form of an arrow shows those of the DADP and a continuous line shows those of acetone. The characteristic signals of each compound (TATP, DADP and acetone) have been obtained by comparison with a previous publication (L. Jensen, P. M. Mortensen, R. Trane, P. Harris, R. W. Berg. Appl. Spectrosc. 63(1), 92-97, 2009).
Figures 8a - 8d: Scanning electron microscope (SEM) photographs of different parts of the Hodei-TATP support according to particular embodiments of the present invention.
Figure 9: Raman spectrum of the base of the Hodei-TATP baits. The characteristic signals of TATP are shown in a continuous line, the form of an arrow shows those of DADP [L. Jensen, P. M. Mortensen, R. Trane, P. Harris, R. W. Berg. Appl. Spectrosc. 63(1), 92-97, 2009] and dashed lines shows those corresponding to the components of the support (cellulose acetate, TiO₂, ZnO and CaO). Identification made based on the information provided by the databases "Renishaw minerals and inorganic materials database" and "Renishaw polymeric materials database".
Figures 10a - 10c: SCAN mode chromatograms performed by means of HS-SPME-GC/MS of the sample of the TATP 1 2 bait used in Example 5 (Figure 10a), sample of the TATP 1 3 bait used in Example 5 (Figure 10b) and sample of the TATP 1 3 bait at the time of its manufacture (July 2019).

### EXAMPLES

### 1. Synthesis of a bait comprising solid TATP adsorbed on a support made of cellulosic material (baits referred to as Hauts-TATP)

To carry out the synthesis of Hauts-TATP baits, 199 µl (2.7 mmol) of 98 % v/v acetone (Panreac, Barcelona, Spain) and 278 µl (2.7 mmol) of 30% v/v hydrogen peroxide (Sigma-Aldrich, Saint Louis, United States) were mixed in a round-bottomed flask and in a 9 °C ice/water bath while the solution was stirred. 37.8 µl (0.675 mmol) of 98% w/w sulfuric acid (Scharlau, Barcelona, Spain) were added for 15 minutes. The reaction was left to stir in the bath for a minimum of 5 hours. After this time, 14 ml of ultrapure water were added to the precipitated TATP obtained after the reaction to be able to filter the TATP through a vacuum system. A glass wool filter (Scharlab, Barcelona, Spain) with a thickness of 47 mm was placed on the Büchner funnel. Supports made of acetylated cellulose forming the baits of the invention were placed on top, and then the suspension of TATP was added onto these supports. Subsequently, cleaning was done with 10 ml of water to remove any trace of acid, and it was left to filter for half an hour to dry the Hauts-TATP baits. The baits obtained were stored in a screw-top glass vial in the freezer until use at -18 °C. The amount of water was determined by thermogravimetry, not being more than 10% by weight with respect to the total weight of the bait (W_{H2O}/W_{BAIT}).

Following the procedure described above, a sufficient amount of TATP was achieved for a maximum of 6 Hauts-TATP baits, these baits containing an amount of 21 mg to 0.7 mg of solid TATP.

The supports made of cellulosic material used for the synthesis described in this section were 6mm Krypton Slim^{®} and 6mm Extra+ Slim^{®} brand tobacco filters (cellulose acetate).

In the following SEM photographs (Figure 6), it can be observed how the solid explosive is adsorbed between the cellulose acetate fibres throughout the entire internal volume of the solid support used.

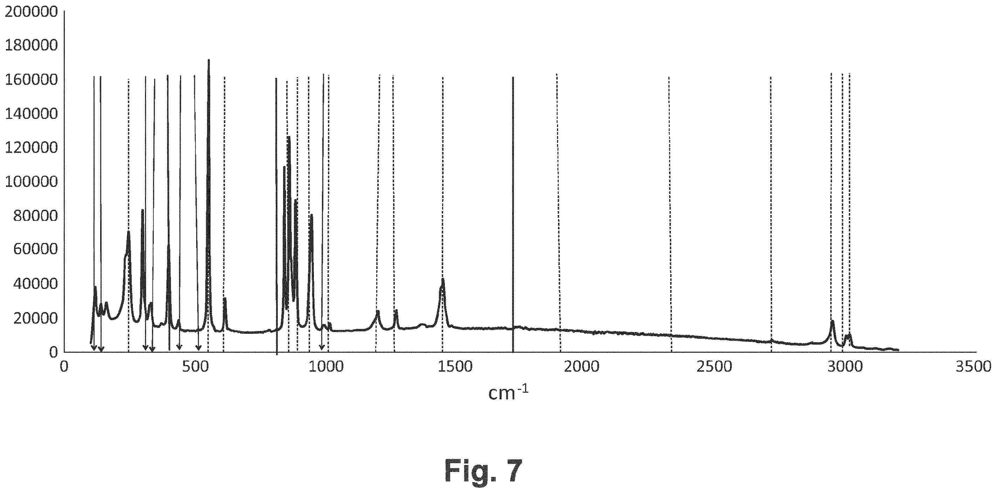

Furthermore, the presence of the explosive in the bait was verified by measuring the base of the bait in which the greatest amount of TATP was deposited in the filtering process. Figure 7 shows the characteristic bands of TATP (Table 2) in the Raman spectrum. As mentioned above, DADP and acetone appear naturally in baits because they are TATP degradation products. The presence of the three mentioned molecules can be observed in the Raman spectrum. The presence of these degradation compounds was also observed in the analysis by means of HS-SPME-GC/MS (Figure 5).

**Table 2. Characteristic bands of the TATP obtained by comparison with a previous publication (L. Jensen, P. M. Mortensen, R. Trane, P. Harris, R. W. Berg. Appl. Spectrosc. 63(1), 92-97, 2009)**

| Wave length /cm-1 |
|---|
| 3014 |
| 3000 |
| 2945 |
| 2720 |
| 2329 |
| 1452 |
| 1276 |
| 1201 |
| 951 |
| 895 |
| 868 |
| 823 |
| 623 |
| 563 |
| 403 |
| 314 |
| 244 |

### 2. Synthesis of a bait comprising TATP vapour adsorbed on a support (baits referred to as Hodei-TATP)

To carry out the synthesis of Hodei-TATP baits, 199 µl (2.7 mmol) of 98 % v/v acetone (Panreac, Barcelona, Spain) and 278 µl (2.7 mmol) of 30% v/v hydrogen peroxide (Sigma-Aldrich, Saint Louis, United States) were mixed in a round-bottomed flask and in a 9 °C ice/water bath while the solution was stirred. 37.8 µl (0.675 mmol) of 98% w/w sulfuric acid (Scharlau, Barcelona, Spain) were added for 15 minutes. The reaction was left to stir in the bath for a minimum of 5 hours. After this time, 14 ml of ultrapure water were added to the precipitated TATP obtained after the reaction to be able to filter the TATP through a vacuum system. A glass wool filter (Scharlab, Barcelona, Spain) with a thickness of 47 mm was placed on the Büchner funnel and the aqueous suspension of TATP was filtered. Subsequently, cleaning was done with 10 ml of water to remove any trace of acid, and it was left to filter for half an hour to dry the solid TATP.

Once the necessary quantity of solid TATP has been obtained by reproducing the procedure described above one or more times, the following was performed to obtain the Hodei-TATP bait: The solid was carefully collected with a spatula without rubbing or hitting. It was gradually transferred to the bottom of a glass tube 120 mm long and 1.2 cm wide. Once the amount of between 6 mg and 200 mg of TATP is placed in a horizontal position in the container, a support made of cellulosic material, in particular 6mm Krypton Slim^{®} and 6mm Extra+ Slim^{®} brand tobacco filters (cellulose acetate), was arranged in the test tube, without touching the solid TATP. In particular, the distance between the solid TATP and the support made of cellulose acetate was kept between 1.5 cm and 3 cm. Subsequently, the test tube was covered with a plastic stopper and left to rest for a period between 7 days and 30 days, at a temperature between 10 °C and 35 °C. After this time, the support made of cellulosic material with TATP vapour was removed from the test tube and kept in a sealed vial until use.

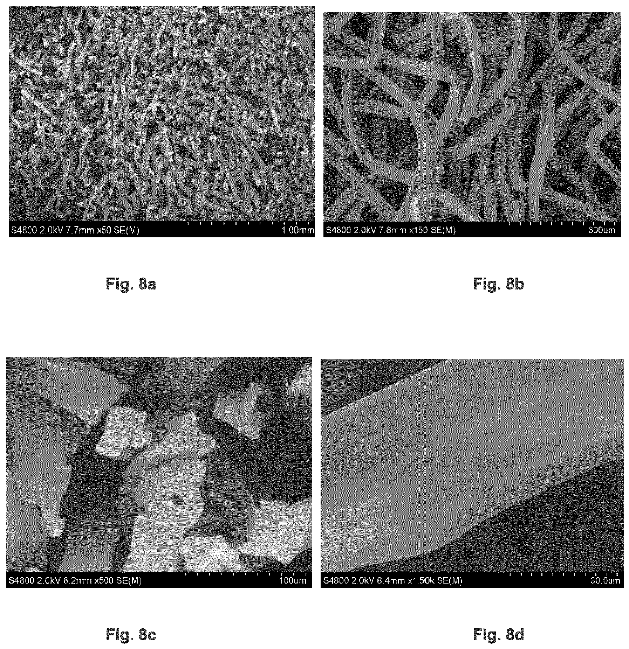

As can be seen in the SEM image (Figures 8a-8d), unlike the Hauts-TATP baits where solid particles were visible in the cellulose acetate fibres (see Figures 6a-6d), in the case of Hodei-TATP baits, no solid particles of the explosive are observed in the fibres. This proves, as explained above, that in the process of making the Hodei-TATP baits there is no redeposition process for the explosive, but rather it is the aroma/vapour of the TATP explosive that remains adsorbed on the cellulosic material itself.

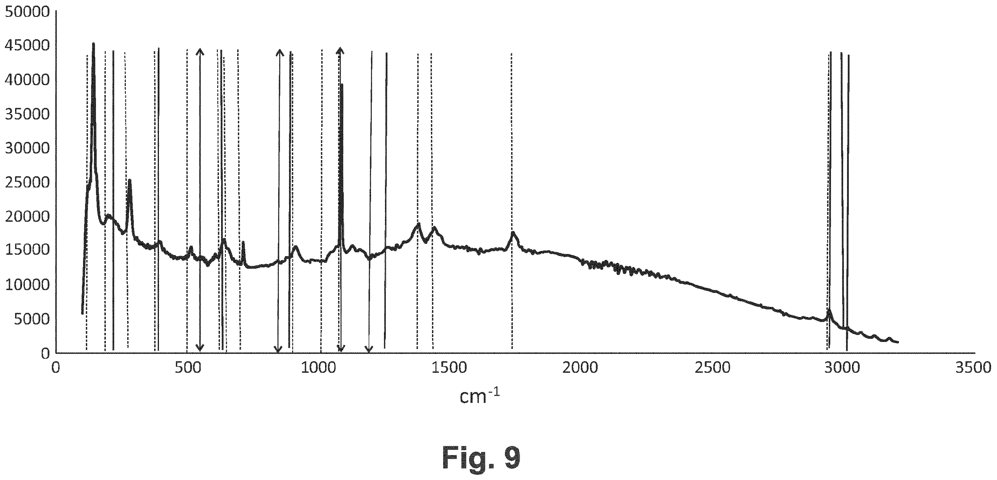

The presence of this TATP aroma in the fibres of the bait could be verified by means of HS-SPME-GC/MS analysis (Figure 5b) and in a less evident way by means of Raman spectroscopy analysis. The presence of TATP in these baits is lower than in the Hauts-TATP baits and this is noted in the Raman spectrum (Figure 9). In this case, the bands corresponding to the components of the solid support are very present, while the signals corresponding to the explosive are more tenuous. Signals corresponding to the degradation products of TATP are also present.

### 3. Use of Hauts-TATP baits for training dogs in the detection of explosives

To demonstrate the efficacy of baits where solid TATP is adsorbed on a cellulosic support, canine training was carried out for two days with the training baits according to the present invention.

Before canine training, several syntheses of the baits of the present invention were carried out as described in section 1 of the examples, and each bait was given a number. The baits were weighed to determine the amount of TATP contained in them. The baits used and the amounts of TATP contained in them are found in the following Table 3.

**Table 3. Weights (in mg) of each of the amounts of TATP explosive in the baits used**

| Baits used in the tests | TATP mg |
|---|---|
| TATP1 1 | 14.4 |
| TATP1 2 | 2.6 |
| TATP1 3 | 15 |
| TATP1 5 | 2.4 |
| TATP2 1 | 0.7 |
| TATP2 2 | 9.8 |
| TATP2 3 | 4.9 |
| TATP2 6 | 20.7 |
| TATP3 2 HCl | 6.62 |
| TATP3 3 HCl | 4.3 |
| TATP4 1 | 15.3 |
| TATP4 3 | 0.9 |
| TATP4 6 | 7 |
| TATP5 2 | 12.3 |
| TATP5 4 | 3.2 |
| TATP5 5 | 5.1 |

Baits TATP3 2 HCl and TATP3 3 HCl were obtained following the procedure previously indicated in section 1 of the examples, with the exception that 27% hydrochloric acid (Tracepure^{®}, Merck, Darmstadt, Germany) was used, maintaining a 3:3:1 molar ratio of acetone: hydrogen peroxide: acid.

Then the baits that would be used for each training test were randomly chosen.

The temperature ranged from 10 °C to 23 °C and the humidity from 83% to 93%. Seven sniffing dogs of different breeds, ages and sex, that had been previously trained in the detection of the explosive in question, were used.

To perform 8 types of easy and short exercises (not more than 5 minutes), 5 different scenarios, both open and closed, were used since outdoor locations better simulate operational conditions, due to the variability of external stimuli, unlike closed scenarios, where conditions are more stable. The locations were: open field, wooden shed with gardening instruments, living room, garage, and the entrance of an office building with a strong ventilation duct.

The training was divided into two blocks: detection and discrimination. The detection block was made to ensure that the dog detects the explosive; in contrast, the discrimination block was made to ensure that the dog can differentiate it from other scents. In this case, as one of the reagents, and also degradation products of TATP, is acetone; it was one of the distractors used. In this same way, the same supports made of acetylated cellulose were used to create the Hauts-TATP baits and the vials where they were stored to ensure that the dogs were looking for the scent of the explosive and not the supports. The training was carried out using positive reinforcement techniques (if the dogs were able to detect the bait, they were given a toy). The exercises performed are listed in the following Table 4.

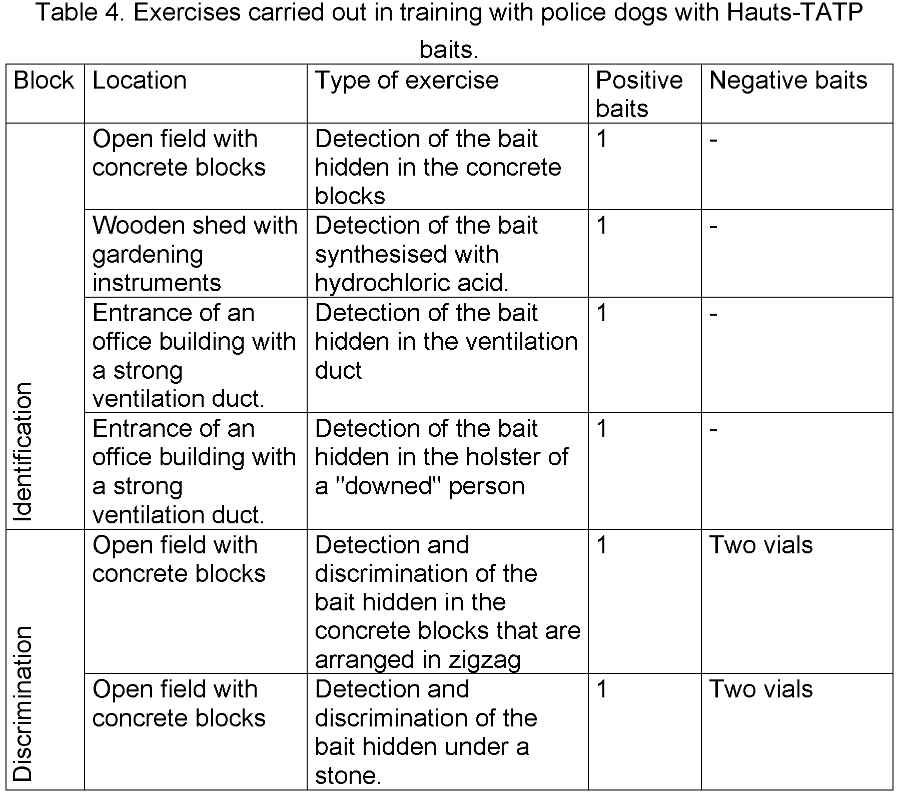

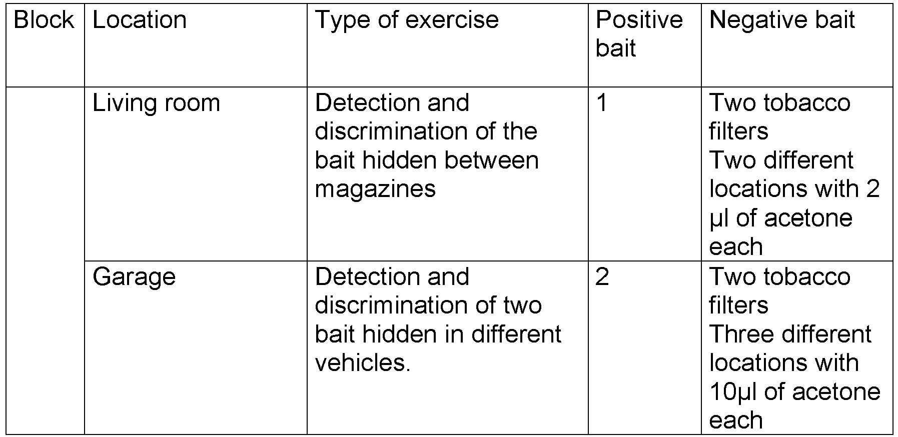

Of the 58 positive baits, 55 were detected correctly, given that there were three false positives (the presence of TATP was wrongly flagged on three occasions), therefore, the sensitivity of dogs to correctly detect TATP was 94.8%.

85 of the 86 distractors were detected correctly, since a negative bait, specifically a vial, was detected as a false negative, thus giving a specificity, or ability to determine correctly when a compound is not 98.8% TATP.

All baits tested worked equally well and the few errors detected (false positives, negatives or non-detections) cannot be attributed to an insufficient amount of TATP in the training baits. In fact, the bait containing the least amount was used in an outdoor open field, being located under a stone, one of the most difficult exercises to perform in training, giving positive results.

### 4. Use of Hodei-TATP baits for training dogs in the detection of explosives

To demonstrate the efficacy of baits (Hodei-TATP) in which solid TATP is adsorbed in vapour form on a support made of cellulosic material, dog training was carried out for two days with the training baits obtained according to the procedure described in section 2 of the examples. In particular, the conditions in which the adsorption of TATP vapour on the cellulosic support made of cellulose acetate took place to obtain the Hodei-TATP baits used in the training sessions are included in Table 5.

**Table 5: Synthesis conditions of Hodei-TATP baits used in canine training**

| Baits | Baits of the same synthesis | ¹Exposure distance/cm | Amount of TATP/mg | Exposure time/days | Synthesis period |
|---|---|---|---|---|---|
| Hodei 1 1 | 2 | 2 | 10.9 | 27 | June |
| Hodei 1 2 | | | | | |
| Hodei 2 1 | 3 | 2.5 | 16.7 | 27 | June |
| Hodei 2 3 | | | | | |
| Hodei 3 3 | 3 | 3 | 30 | 26 | July |

| | | | | | |
|---|---|---|---|---|---|
| ¹The syntheses of the Hodei-TATP baits have been carried out in a tube 12 cm long and 1.2 cm wide. | | | | | |

These tests with Hodei baits were carried out by 4 dogs, as shown in Table 6. Training was performed at 18 °C and 93% humidity, using positive reinforcement techniques (if the dogs were able to detect the bait, they were given a toy). There were two false positives, giving a selectivity of 90.0%. Only one discriminant was wrongly flagged as a false negative, giving a specificity of 97.7%.

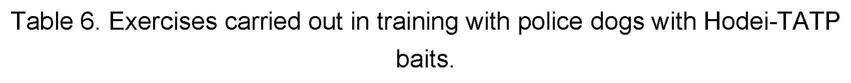

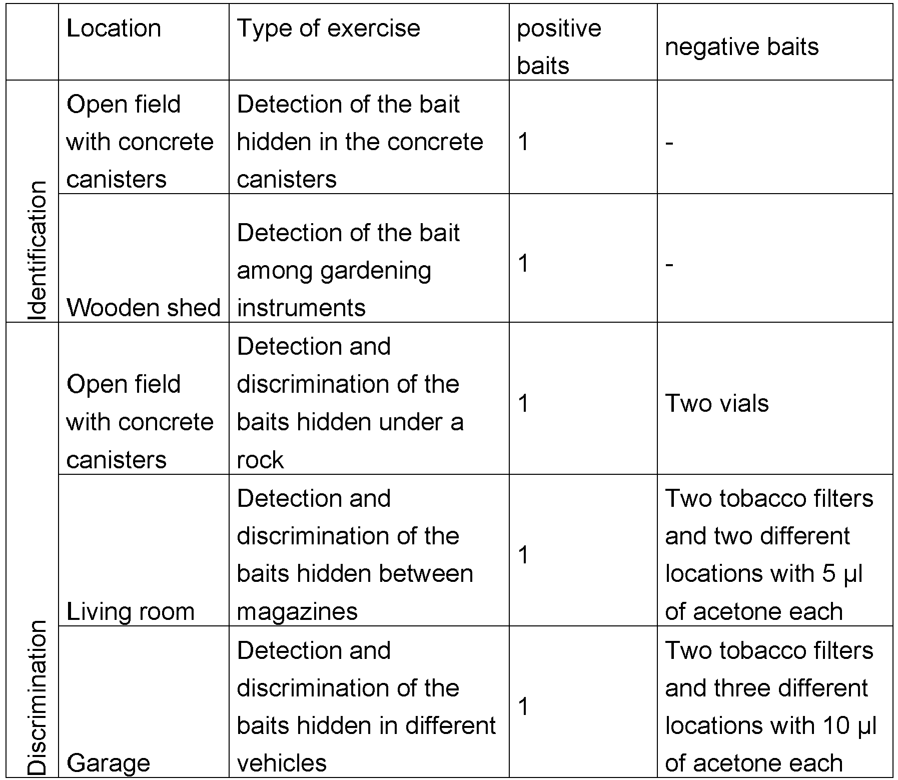

### 5. Demonstration of the durability of TATP in the bait with a support made of acetylated cellulose

### STUDY 5.1

This first study consisted of training designed for dogs trained in the detection of TATP from the canine unit of the Navarre Provincial Police carried out on 15/07/2021. To carry out this training, 2 baits were used for which both the TATP synthesis and the production of the bait had taken place 24 months previously (TATP 1 2 and TATP 1 3 baits). In both cases, both the synthesis of the TATP and the manufacture of the bait took place according to the procedure described herein.

During these 15 months these TATP 1 2 and TATP 1 3 baits have been used by the canine unit of the ERTZAINTZA with a frequency of 1 time every 2 months (about 12 times), for at least 1 hour of exposure and training. This means that each of the reused baits that have been used in this experiment have been used for at least 12 hours in multiple training spaces: open spaces, rooms, cars, people, etc.

To demonstrate the efficacy of reused baits, dog training was performed for one day with the training baits described above. The baits used in this test and the amounts of TATP contained in them are found in the following Table 7.

**Table 7. Weights (in mg) of each of the amounts of TATP explosive in the baits used in this test when they were carried out in July 2019**

| Baits used in the 2021 tests | TATP weight in mg |
|---|---|
| TATP 1 2 | 2.6 |
| TATP 1 3 | 15.0 |

All tests were carried out on the same day. The temperature ranged from 18 to 20 °C and the humidity from 83% to 93%. Five sniffing dogs of different breeds, ages and sex, that had been previously trained in the detection of the explosive in question, were used.

To perform 3 types of easy and short exercises (not more than 5 minutes), 3 different scenarios, both open and closed, were used since outdoor locations better simulate operational conditions, due to the variability of external stimuli, unlike closed scenarios, where conditions are more stable. The 3 locations were: open field, living room and garage.

The training was divided into two blocks: detection and discrimination. The detection block was made to ensure that the dog detects the explosive; in contrast, the discrimination block was made to ensure that the dog can differentiate it from other scents. In this case, the same supports made of acetylated cellulose used to produce the baits of the present invention and the vials where they were stored were used to ensure that the dogs were looking for the scent of the explosive and not the supports. The training was carried out using positive reinforcement techniques (if the dogs were able to detect the bait, they were given a toy). The exercises performed are listed in the following Table 8.

**Table 8: Exercises carried out in training with police dogs with Hauts-TATP baits.**

| Block | Location | Type of exercise | Positive baits | Negative baits |
|---|---|---|---|---|
| Identification | Open field | Point-to-point bait detection | 1 | - |
| | Living room | Detection of a bait hidden in the ground and/or pots | 2 | - |
| Discrimination | Open field | Point-to-point bait detection | 1 | 2 (empty vial and white filter) |
| | Garage | Detection and discrimination of 2 baits hidden in two vehicles | 2 | 2 (blank filters) |
| | Living room | Detection of a bait hidden in the ground and/or pots | 1 | 1 (blank filter) |

Of the 35 positive baits, 31 were detected correctly, given that there were three false positives (the presence of TATP was wrongly flagged on three occasions), therefore, the sensitivity of dogs to correctly detect TATP was 88.6 %. 24 of the 25 distractors were detected correctly, since a negative bait, specifically a support filter, was detected as a false negative, thus giving a specificity, or ability to determine correctly when a compound is not 96 % TATP. As can be seen, there is a decrease in the sensitivity and specificity values of the reused priming charge, but they are still suitable for reinforcing and training dogs.

### STUDY 5.2

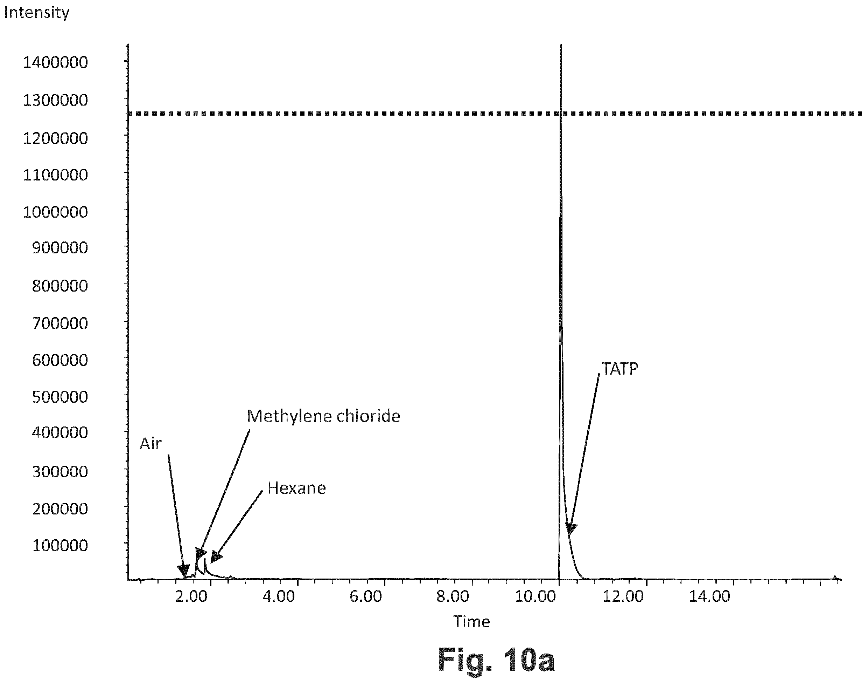

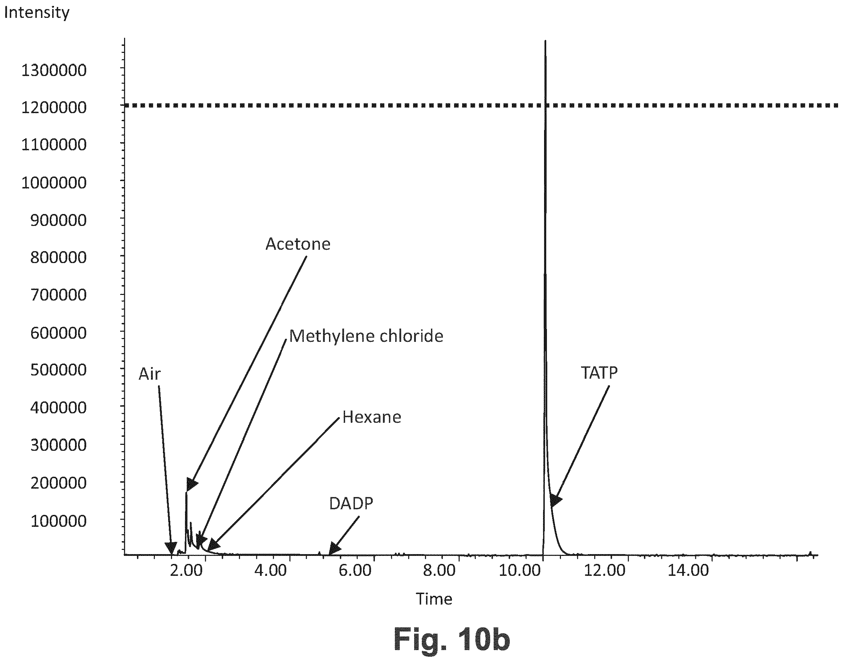

The second study consisted of HS-SPME-GC/MS analysis of the two baits used for study 5.1. Figures 10a - 10b show the chromatograms of the two baits that were synthesised two years ago and that have been used over this time by the canine unit of the ERTZAINTZA for training their dogs. In particular, as can be seen (Figure 10b), an intense signal of the explosive can be seen in the support of the TATP 1 3 bait (t_{R.}: 10.3 min) characterised and identified as TATP when compared with the mass spectral library. In the same way, it is possible to find the same signal (t_{R.}: 10.3 min) on the support of the other sample used, TATP 1 2 (Figure 10a). In addition to the signal corresponding to the TATP, it is possible to observe signals identified as acetone, dichloromethane, hexane or DADP (see Figures 10a and 10b) upon comparing their mass spectra against the spectral library. These compounds may be related to the degradation of TATP itself (in the case of acetone or DADP) or to the contamination of the supports during use over these two years.

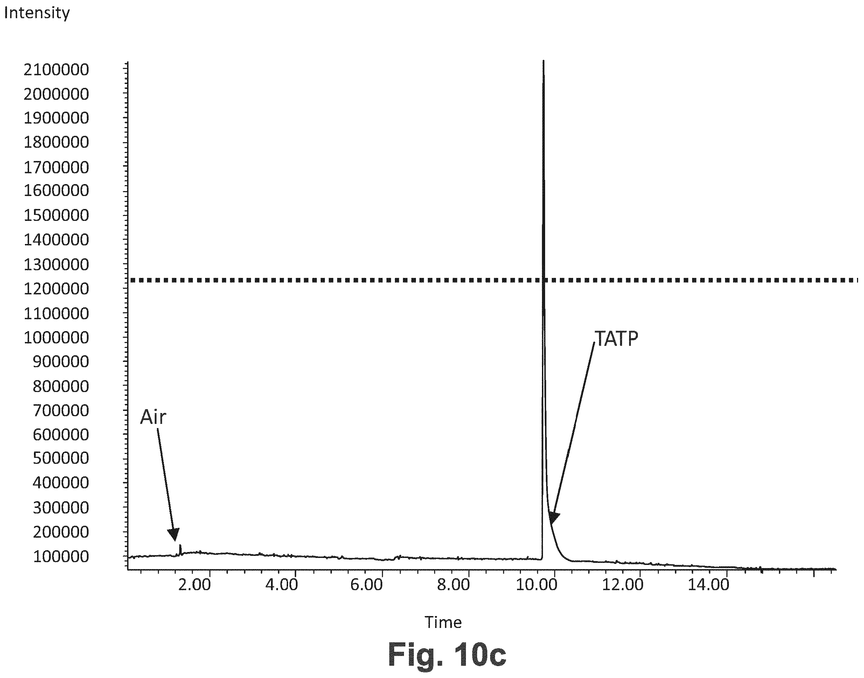

The degradation of TATP and its loss can be clearly seen if the signal intensity of the baits at this present moment (July 2021) is compared with the signal intensity at the time these baits were created (July 2019). Figure 10c shows the chromatogram of the TATP 1 3 bait at the time of its manufacture in July 2019. As can be seen, the TATP signal intensity is almost twice that obtained by the two baits two years later (see the dashed line that corresponds to the maximum of the signals in 2021). It is also observed that the bait does not have as many interference signals because it had just been manufactured.

For all these reasons, in this double study it has been empirically verified that:
- An analytical signal has been found in the analyses of the volatile components of the reused bait by means of HS-SPME-GC/MS that has been unequivocally identified as TATP.
- After 12 hours of use and exposure of these baits and after two years of storage at 4 °C, the presence of TATP is observed although the signal intensity of this compound has decreased by almost half when compared with the results obtained at the time of manufacture.
- Baits continue to offer levels of specificity and selectivity that allow dog training.

## Claims

1. A bait for training in the detection of explosives, **characterised in that** it comprises a maximum of 0.05 g of triacetone triperoxide (TATP) adsorbed on a solid support comprising a cellulosic material, wherein the bait preferably comprises a maximum of 10% by weight of water, said percentage being expressed by weight with respect to the total weight of the bait, wherein the cellulosic material is acetylated cellulose.

2. The bait according to claim 1, wherein the solid support is a single piece.

3. The bait according to claim 2, wherein the solid support is a single piece having a volume between 100 mm³ and 2300 mm³.

4. The bait according to any one of claims 1 to 3, wherein the TATP content is 50 mg to 0.5 mg of TATP.

5. The bait according to any one of claims 1 to 3, wherein **TATP** is in the form of a **TATP** vapour adsorbed on the support.

6. A process for obtaining a bait for training in the detection of explosives as described in any one of claims 1 to 4, **characterised in that** it comprises:
a) obtaining an aqueous suspension of **TATP,** and
b) adsorbing the **TATP** from the aqueous suspension of step a) on a solid support comprising a cellulosic material, wherein the cellulosic material is acetylated cellulose.

7. The process according to claim 6, wherein the aqueous suspension of **TATP** is obtained by means of a process comprising the steps of:
a-i) mixing acetone and hydrogen peroxide at a temperature of 3 to 16 °C;
a-ii) adding acid to the mixture obtained in a-i) for a time of 10 to 30 minutes;
a-iii) stirring the mixture of a-ii) for at least 5 hours, obtaining triacetone triperoxide (TATP); and
a-iv) suspending the triacetone triperoxide (TATP) of step a-iii) in water, obtaining the aqueous suspension of TATP;
wherein the stoichiometric ratio of acetone:hydrogen peroxide:acid is 3:3:1, and the amount of acetone is less than 10 mmol.

8. A process for obtaining the bait for training in the detection of explosives as described in any one of claims 1 to 3 and 5, comprising keeping solid TATP and a solid support comprising a cellulosic material in the same closed container for a minimum period of 7 days, without contact between the two solids, wherein the cellulosic material is acetylated cellulose.

9. The process according to claim 8, **characterised in that** the solid TATP is obtained by means of a process comprising the steps of:
i) mixing acetone and hydrogen peroxide at a temperature of 3 to 16 °C; and
ii) adding acid to the mixture obtained in a) for a time of 10 to 30 minutes;
iii) stirring the mixture of b) for at least 5 hours, obtaining solid triacetone triperoxide (TATP);
wherein the stoichiometric ratio of acetone:hydrogen peroxide:acid is 3:3:1, and the amount of acetone is less than 10 mmol.

10. The obtaining process according to claim 9, wherein said process comprises the following additional steps:
iv) suspending the triacetone triperoxide (TATP) of step iii) in water, obtaining the aqueous suspension of TATP; and
v) filtering the suspension of step iv).

11. The process according to any one of claims 7, 9 to 10, wherein the amount of acetone is equal to or less than 2.7 mmol.

12. The process according to any one of claims 7, 9 to 11, wherein the acid of step a-ii) or ii) is selected from the group consisting of sulfuric acid, hydrochloric acid and nitric acid.

13. Use of the TATP bait described in any one of claims 1 to 5 for training dogs, preferably police dogs, in the detection of TATP explosives.

14. The use of the bait according to claim 13, wherein the desorption of TATP from the cellulosic material takes place at atmospheric pressure and a temperature between 10 °C and 35 °C, and wherein the cellulosic material is acetylated cellulose.

## Patentansprüche

1. Köder für die Ausbildung in der Sprengstofferkennung, **dadurch gekennzeichnet, dass** er maximal 0,05 g Triacetontriperoxid (TATP) enthält, das an einem festen Träger adsorbiert ist, der ein Cellulosematerial enthält, wobei der Köder vorzugsweise maximal 10 Gew.-%Wasser enthält, wobei der Prozentsatz als Gewichtsprozent in Bezug auf das Gesamtgewicht des Köders ausgedrückt wird, wobei das Cellulosematerial acetylierte Cellulose ist.

2. Köder nach Anspruch 1, wobei der feste Träger aus einem einzigen Stück ist.

3. Köder nach Anspruch 2, wobei der feste Träger aus einem einzigen Stück mit einem Volumen zwischen 100 mm³ und 2300 mm³ ist.

4. Köder nach einem der Ansprüche 1 bis 3, wobei der TATP-Gehalt 50 mg bis 0,5 mg TATP beträgt.

5. Köder nach einem der Ansprüche 1 bis 3, wobei das TATP in Form eines an den Träger adsorbierten TATP-Dampfes vorliegt.

6. Verfahren zur Herstellung eines Köders für die Ausbildung in der Sprengstofferkennung nach einem der Ansprüche 1 bis 4, **dadurch gekennzeichnet, dass** es umfasst:
a) Gewinnen einer wässrigen TATP-Suspension, und
b) Adsorbieren des TATP aus der wässrigen Suspension von Schritt a) an einem festen Träger, der ein Cellulosematerial enthält, wobei das Cellulosematerial acetylierte Cellulose ist.

7. Verfahren nach Anspruch 6, wobei die wässrige TATP-Suspension durch ein Verfahren gewonnen wird, das diese Schritte umfasst:
a-i) Mischen von Aceton und Wasserstoffperoxid bei einer Temperatur von 3 bis 16 °C;
a-ii) Zugabe von Säure zu der in a-i) erhaltenen Mischung für eine Dauer von 10 bis 30 Minuten;
a-iii) Rühren der Mischung aus a-ii) für mindestens 5 Stunden, woraus Triacetontriperoxid (TATP) gewonnen wird; und
a-iv) Suspendieren des Triacetontrperoxids (TATP) aus Schritt a-iii) in Wasser, woraus die wässrige TATP-Suspension erhalten wird;
wobei das stöchiometrische Verhältnis von Aceton: Wasserstoffperoxid: Säure 3:3:1 beträgt und die Menge an Aceton weniger als 10 mmol beträgt.

8. Verfahren zur Herstellung eines Köders für die Ausbildung in der Sprengstofferkennung, wie in einem der Ansprüche 1 bis 3 und 5 beschrieben, umfassend das Aufbewahren von festem TATP und einem festen Träger, der ein zellulosehaltiges Material enthält, in demselben geschlossenen Behälter für einen Mindestzeitraum von 7 Tagen ohne Kontakt zwischen den beiden Feststoffen, wobei das zellulosehaltige Material acetylierte Zellulose ist.

9. Verfahren nach Anspruch 8, **dadurch gekennzeichnet, dass** das feste TATP durch ein Verfahren gewonnen wird, das die Schritte umfasst:
i. Mischen von Aceton und Wasserstoffperoxid bei einer Temperatur von 3 bis 16 °C; und ii) Zugabe von Säure zu der in a) gewonnenen Mischung für eine Dauer von 10 bis 30 Minuten;
ii. Rühren der Mischung aus b) für mindestens 5 Stunden, woraus Triacetontriperoxid (TATP) erhalten wird;
iii. wobei das stöchiometrische Verhältnis von Aceton: Wasserstoffperoxid:Säure 3:3:1 beträgt und die Menge an Aceton weniger als 10 mmol beträgt.

10. Verfahren zum Gewinnen nach Anspruch 9, wobei das Verfahren die folgenden zusätzlichen Schritte umfasst:
iv. Suspendieren des Triacetontriperoxids (TATP) aus Schritt iii) in Wasser, woraus die wässrige TATP-Suspension gewonnen wird; und
v. Filtrieren der Suspension aus Schritt iv).

11. Verfahren nach einem der Ansprüche 7, 9 bis 10, wobei die Menge an Aceton gleich oder weniger als 2,7 mmol beträgt.

12. Verfahren nach einem der Ansprüche 7, 9 bis 11, wobei die Säure aus Schritt a-ii) oder ii) ausgewählt ist aus der Gruppe bestehend aus Schwefelsäure, Salzsäure und Salpetersäure.

13. Verwendung des in einem der Ansprüche 1 bis 5 beschriebenen TATP-Köders zur Ausbildung von Hunden, vorzugsweise von Polizeihunden, für das Erkennen von TATP-Sprengstoffen.

14. Verwendung des Köders nach Anspruch 13, wobei die Desorption von TATP aus dem Cellulosematerial bei Atmosphärendruck und einer Temperatur zwischen 10°C und 35°Cstattfindet und wobei das Cellulosematerial acetylierte Cellulose ist.

## Revendications

1. Appât pour l'entraînement dans la détection d'explosifs, **caractérisé en ce qu'**il comprend au maximum 0,05 g de triperoxyde de triacétone (TATP) adsorbé sur un support solide comprenant un matériau cellulosique, lequel appât comprend de préférence au maximum 10 % en poids d'eau, ledit pourcentage étant exprimé en poids par rapport au poids total de l'appât, dans lequel le matériau cellulosique est une cellulose acétylée.

2. Appât selon la revendication 1, dans lequel le support solide consiste en un seul morceau.

3. Appât selon la revendication 2, dans lequel le support solide consiste en un seul morceau ayant un volume compris entre 100 mm³ et 2 300 mm³.

4. Appât selon l'une quelconque des revendications 1 à 3, dans lequel la teneur en TATP est de 50 mg à 0,5 mg de TATP.

5. Appât selon l'une quelconque des revendications 1 à 3, dans lequel le TATP est sous la forme d'une vapeur de TATP adsorbée sur le support.

6. Procédé pour obtenir un appât pour l'entraînement dans la détection d'explosifs tel que décrit dans l'une quelconque des revendications 1 à 4, **caractérisé en ce qu'**il comprend :
a) l'obtention d'une suspension aqueuse de TATP, et
b) l'adsorption du TATP depuis la suspension aqueuse de l'étape a) sur un support solide comprenant un matériau cellulosique, lequel matériau cellulosique est une cellulose acétylée.

7. Procédé selon la revendication 6, dans lequel la suspension aqueuse de TATP est obtenue au moyen d'un procédé comprenant les étapes de :
a-i) mélange d'acétone et de peroxyde d'hydrogène à une température de 3 à 16 °C ;
a-ii) addition d'acide au mélange obtenu en a-i) pendant un temps de 10 à 30 minutes ;
a-iii) agitation du mélange de a-ii) pendant au moins 5 heures, obtention de triperoxyde de triacétone (TATP) ; et
a-iv) mise en suspension du triperoxyde de triacétone (TATP) de l'étape a-iii) dans de l'eau, obtention de la suspension aqueuse de TATP ;
dans lequel le rapport stœchiométrique acétone/peroxyde d'hydrogène/acide est de 3/3/1, et la quantité d'acétone est inférieure à 10 mmol.

8. Procédé pour obtenir l'appât pour l'entraînement dans la détection d'explosifs tel que décrit dans l'une quelconque des revendications 1 à 3 et 5, comprenant le maintien de TATP solide et d'un support solide comprenant un matériau cellulosique dans le même récipient fermé pendant une période minimale de 7 jours, sans contact entre les deux solides, dans lequel le matériau cellulosique est une cellulose acétylée.

9. Procédé selon la revendication 8, **caractérisé en ce que** le TATP solide est obtenu au moyen d'un procédé comprenant les étapes de :
i) mélange d'acétone et de peroxyde d'hydrogène à une température de 3 à 16 °C ; et
ii) addition d'acide au mélange obtenu en a) pendant un temps de 10 à 30 minutes ;
iii) agitation du mélange de b) pendant au moins 5 heures, obtention de triperoxyde de triacétone (TATP) solide ;
dans lequel le rapport stœchiométrique acétone/peroxyde d'hydrogène/acide est de 3/3/1, et la quantité d'acétone est inférieure à 10 mmol.

10. Procédé d'obtention selon la revendication 9, lequel procédé comprend les étapes additionnelles suivantes :
iv) mise en suspension du triperoxyde de triacétone (TATP) de l'étape iii) dans de l'eau, obtention de la suspension aqueuse de TATP ; et
v) filtration de la suspension de l'étape iv).

11. Procédé selon l'une quelconque des revendications 7, 9 et 10, dans lequel la quantité d'acétone est égale ou inférieure à 2,7 mmol.

12. Procédé selon l'une quelconque des revendications 7 et 9 à 11, dans lequel l'acide de l'étape a-ii) ou ii) est choisi dans le groupe constitué par l'acide sulfurique, l'acide chlorhydrique et l'acide nitrique.

13. Utilisation de l'appât au TATP décrit dans l'une quelconque des revendications 1 à 5 pour entraîner des chiens, de préférence des chiens policiers, à la détection d'explosifs au TATP.

14. Utilisation d'un appât selon la revendication 13, dans laquelle la désorption de TATP à partir du matériau cellulosique a lieu sous la pression atmosphérique et à une température comprise entre 10 °C et 35 °C, et dans laquelle le matériau cellulosique est une cellulose acétylée.
